# EUROPEAN PATENT SPECIFICATION

(11) **EP 1 709 768 B1**
(45) Date of publication and mention of the grant of the patent: **18.11.2015**
(21) Application number: 05702587.6
(22) Date of filing: 05.01.2005
(51) Int. Cl.: H04W 4/18, A61B 5/00, H04W 84/12, H04W 88/08

(54) **INTERMEDIATE DISTRIBUTION FRAME (IDF) FOR MEDICAL DATA BY USING A SMART ETHERNET/IP EMULATING DETECTION AP**
ZWISCHENVERTEILUNGSRAHMEN (IDF) FÜR MEDIZINISCHE DATEN DURCH VERWENDUNG VON INTELLIGENTEM ETHERNET/IP-EMULATIONS-DETEKTIONS-AP
REPARTITEUR INTERMEDIAIRE POUR DONNEES MEDICALES UTILISANT UN POINT D'ACCES D'EMULATION INTELLIGENT ETHERNET/IP

(30) Priority: 15.01.2004 US 536751 P
(43) Date of publication of application: 11.10.2006
(73) Proprietor: Koninklijke Philips N.V., 5656 AE Eindhoven (NL)
(72) Inventor: WITTENBER, Jan, Cleveland, OH 44143 (US); ROSNOV, Brian, Cleveland, OH 44143 (US)
(74) Representative: Verweij, Petronella Danielle
(86) International application number: PCT/IB2005/050057
(87) International publication number: WO 2005/071895

(56) References cited:
- WO-A-02/11397
- US-A- 5 944 659
- US-B1- 6 616 606
- US-B1- 6 659 947
- MEDICAL INFORMATION BUS (MIB) COMMITTEE OF THE IEEE ENGINEERING IN MEDICINE AND BIOLOGY SOCIETY: "IEEE Std 1073-1996, IEEE Standard for Medical Device Communications?Overview and Framework" IEEE STANDARD, 3 October 1996 (1996-10-03), pages 1-19, XP002324852 NEW YORK, USA
- STEVE WARREN, JIANCHU YAO, RYAN SCHMITZ, LUKE NAGL: "Wearable Telemonitoring Systems Designed with Interoperability in Mind" PROCEEDINGS OF THE 25TH ANNUAL INTERNATIONAL CONFERENCE OF THE IEEE EMBS, 17 September 2003 (2003-09-17), pages 3736-3739, XP002324635 CANCUN, MEXICO
- JIANCHU YAO, RYAN SCHMITZ, STEVE WARREN: "A Wearable Standards-Based Point-of-Care System for Home Use" PROCEEDINGS OF THE 25TH ANNUAL INTERNATIONAL CONFERENCE OF THE IEEE EMBS, 17 September 2003 (2003-09-17), pages 3732-3735, XP002324636 CANCOON, MEXICO
- S. CASNER; CISCO SYSTEMS; V. JACOBSON; CISCO SYSTEMS: "RFC 2508, Compressing IP/UDP/RTP Headers for Low-Speed Serial Links" INTERNET ENGINEERING TASK FORCE (IETF) INTERNET SOCIETY (ISOC), February 1999 (1999-02), pages 1-25, XP015008291 GENEVA, SWITZERLAND

## Description

### BACKGROUND OF THE INVENTION

### 1. Field of the Invention

The present invention relates to wireless based and Ethernet-Internet capable Access Point (AP) virtual medical devices, such as bedside monitors. More particularly, the present invention relates to an improvement in wireless medical telemetry systems (WMTS) transmission involving Access Point emulation.

### 2. Description of the Related Art

There are known communication systems, such as wireless medical telemetry systems (WMTS), that are used to monitor patient physiological parameters, such as cardiac signals, over a relatively short distance via radio-frequency between the patient, who is wearing a transmitter, and a central monitoring site or station. The FCC has broadened the definition of WMTS to include the measurement and recording of physiological parameters and other patient related information via radiated bi-directional or uni-directional electromagnetic signals, and has limited WMTS users to authorized healthcare providers, including doctors, healthcare facilities, hospitals and other medical providers.

DECT (Digital Enhanced Cordless Telecommunication standard), which was originally adapted in Europe as a standard for replacing various analog and digital standards for items such as cordless telephones. DECT, which is a microcellular, digital mobile radio network that can be used for high subscribers densities, is well suited for use in buildings, particularly in hospitals where information must be exchanged quickly and accurately among non-stationary persons, such as doctors, nurses, patients, interns, specialists, etc.. DECT is also used in the United States with certain variations of standards adopted in Europe.

In the art, a wireless LAN bridge is considered to be a device that connects two networks that may use the same (or a different) Data Link Layer protocol (for example, layer 2 of the OSI model). LAN bridges have ports that connect two or more LANs that would otherwise be separate. One of the ports of the bridge receives packets and re-transmits them on another port. In general, a bridge does not start transmission until it receives a complete packet.

Bridges, as compared with Access Points, (which connect multiple users to each other on a wireless LAN and to a wired network) are typically less expensive than Access Points as their primary function is to connect other networks. Access Points often require functions not required by bridges because APs provide authentication, de-authentication, association, disassociation, re-association, distribution, MAC service data unit delivery, integration, and privacy services. There is some overlap, for example, in the case of a WLAN bridge adapted to interface with an Ethernet network directly to a particular access point AP, whereby a WLAN bridge plugs into the Ethernet network and uses 802.11 to communicate with an Access Point that is within range.

In recent years, in an attempt to reduce bandwidth requirements, WMTS-DECT-based and Ethernet capable Access Points (APs) have been operating as a WLAN-LAN bridges, thereby shifting the emulation of upper layer functions to the low end devices, which pay the price in terms of bandwidth and power consumption.

Document US 6,616,606 B1 for example discloses a medical telemetry system with wireless patent monitors configured to monitor a patient by collecting vital signs data from the patient, and central stations that receive the vital signs and process them further. The central stations are part of an Ethernet based Local Area Network. The system comprises Access Points connected to the Ethernet on the one hand and, on the other hand, configured to communicate with the wireless patent monitors using a variety of different wireless transmission technologies, e.g. as described under the IEEE 802.11 standard (WLAN) or the IEEE 802.15 standard.

For example, state of the art APs, especially "off-the-shelf" (OTS) APs, operate as WLAN-LAN bridges that cause the above mentioned services to be burdened on the low end devices. Present proprietary technology attempts to solve the problem but requires a limited scale AP and requires a cost-performance ineffective "Gateway" to provide the bridged gateway functionality. Moreover, the use of a bi-directional, cellular wireless technology such as DECT still does not reach significant cost-effectiveness. There have been other failed attempts to moves the AP emulation to concentrators, which then become very complex to manage and scale. Alternatively, emulation burdens can be placed on a device-connected emulating transceiver, which not only fails to improve scalability but increases (rather than decreases) per-bed costs. Thus, there is a need in the art to overcome some of the problems previously mentioned.

### SUMMARY OF THE INVENTION

The object is solved by the features of the independent claims. Further embodiments are subject of the dependent claims.

The present invention provides for a highly efficient, scaleable, cost-effective, proprietary implementations, that would likely be based on industry standards such as, for example, ISO 11073/IEEE 1173.

According to one embodiment of the present invention, a wireless WMTS, DECT-based and Ethernet capable Access Point (AP) emulates a virtual medical device (VMD), so that overall bandwidth resulting from Ethernet and Internet compatibility is reduced to the extent that scalability and throughput is significantly improved over conventional APs. One of the many advantages of the present invention is that existing PWD (Patient Wearable Devices) can continue to operate in their current mode and communicate with the Access Point, so that customers can integrate newer PWDs compatible with the present invention while still being able to use their existing PWDs. The new PWDs, many of which will not need any conversion software because the Access Point is performing the emulation, will have reduced bandwidth and power requirements as compared to the older PWDs.

### BRIEF DESCRIPTION OF THE DRAWINGS

Figs. 1A and 1B illustrate a Data protocol model of communication between a convention wireless Patient Wearable Device (PWD) and a remote site attached to a wired LAN via an emulating Access Point according to the present invention.
Figs. 2A and 2B are an extended Data Protocol Model according to the present invention showing the Gateway Translation Function module when Patient Wearable Devices take advantage of the conversion capabilities of the emulating Access Point according to the present invention.
Fig. 3 illustrates a method of operation according to the present invention.

### DETAILED DESCRIPTION OF THE INVENTION

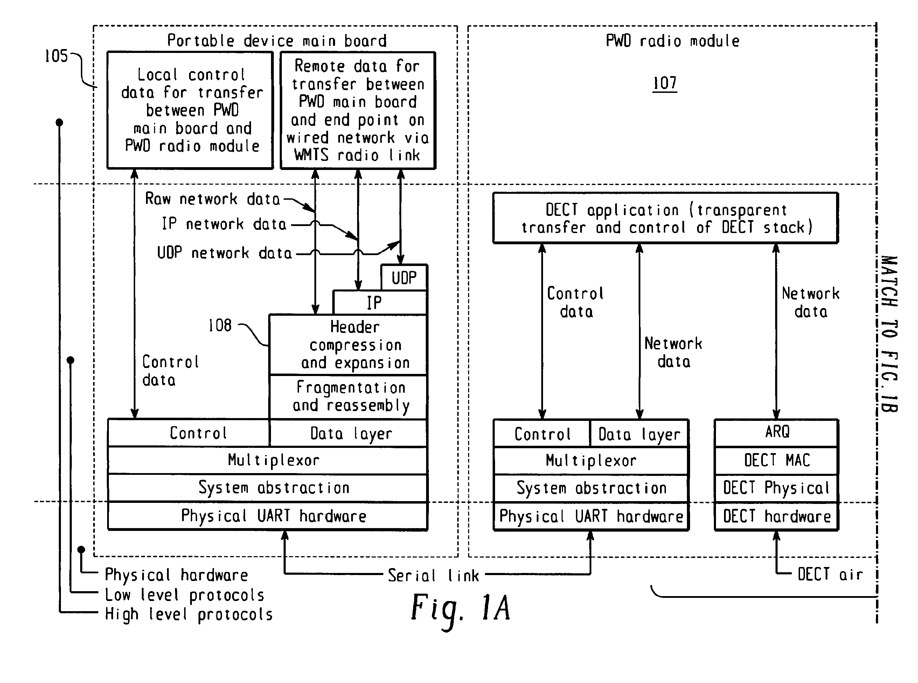

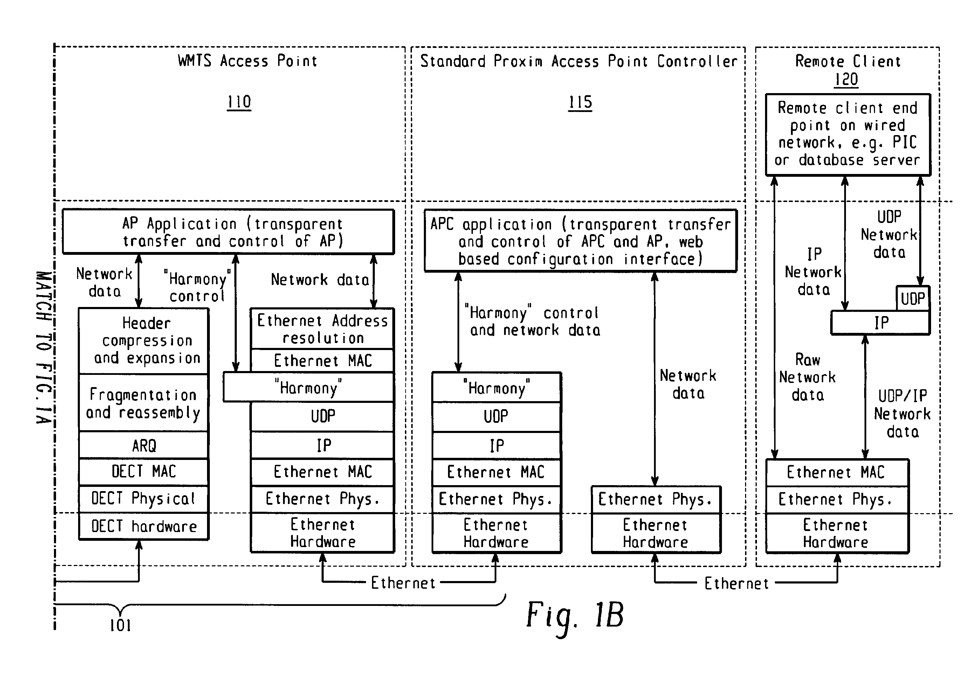

Figs. 1A and 1B illustrate a model of a Data protocol model with a conventional Patient Wearable Device communicating with a remote site on a wired remote LAN via an emulating Access Point, according to the present invention.

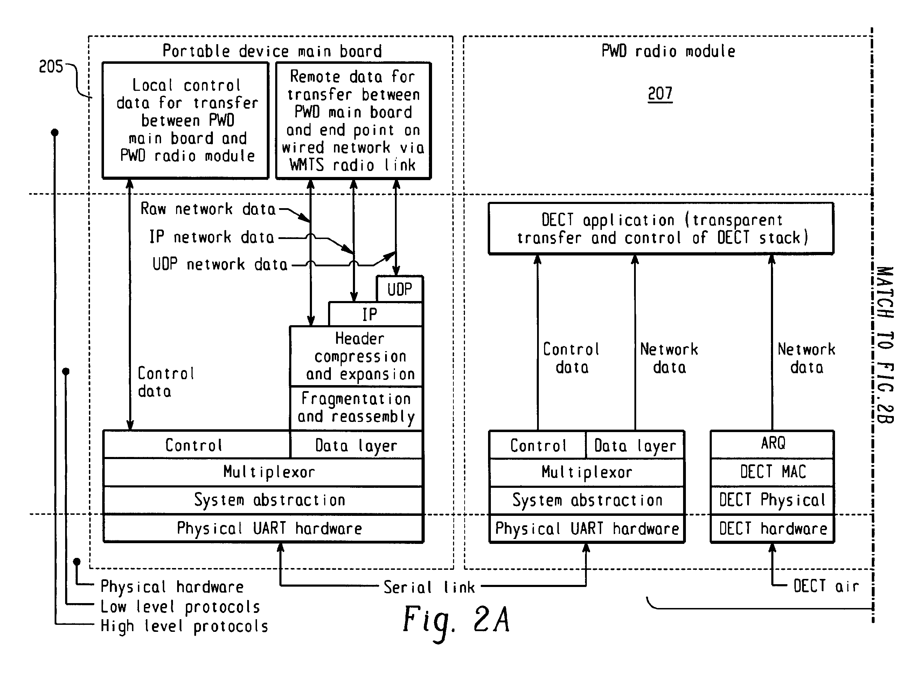

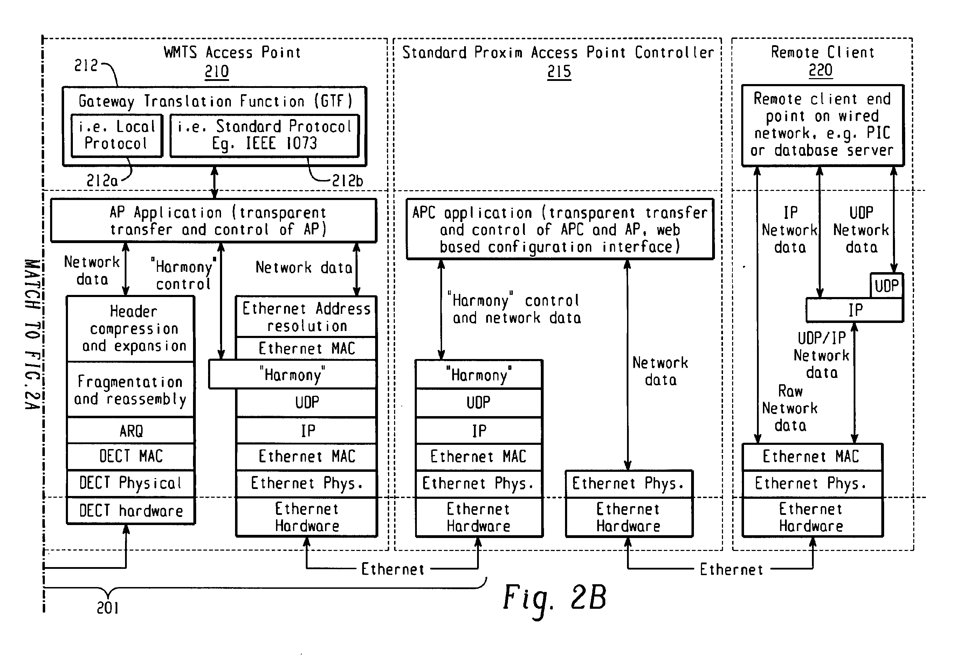

In order to appreciate the advantages of the present invention, it is necessary to some describe some of the components and the functions of the Data Protocol Model shown in Figs. 1A and 1B, as many of these components and functions are operative in the Data Protocol Model according to the present invention (and shown in Figs. 2A and 2B). The PWD in Fig. 1A is conventional in the sense that it utilizes a great deal of bandwidth and power. However, the PWD in Fig. 2A is different in that any conversion/emulation actions are made at the Access Point, so that the wireless communication is transparent to the wired LAN, and the remote client thinks that the PWD is merely another node attached to the LAN. Thus the PWD's in Fig. 2A, while appearing to be the same as the one on Fig. 1A, differ in that they use less power and require less bandwidth than current PWDs. Yet to the remote client, the PWDs appear to be the same (a wired device) because the Access Point takes over the emulation. Thus, future WLANS may have both types of PWDs. However, it is expected that PWD manufacturers would not continue to duplicate circuitry that has now been made unnecessary by the nature of the emulating Access Point, as the PWDs could potentially substantially reduce their costs, as well as their size and required bandwidth use.

The protocol stack 105 provides data transport between the portable device (PWD or PBM) and a remote end point (such as a PIC) or a central database server. The protocol stack allows for data to be returned in the reverse direction at the same rate as sent in the forward direction. In addition, local control messages can be multiplexed across the various communication links. These protocols are split into three area, upper, lower and physical. The back haul system only has knowledge of the lower and physical layers.

A typical data transfer sequence from the portable device 105 though the WMTS 110 to the remote client 120 is as follows:
(1) Data is passed to the lower layer protocols for transport over the UDP (User Datagram Protocol), IP (Internet Protocol) or raw Ethernet data streams. UDP is a lightweight transport normally built on top of that enhances performance from IP by not implementing some of the features of a heavyweight protocol, for example, by allowing individual packets to be dropped without retry, and/or for the packets to be received in a different order than transmitted.
(2) The header compression sub-module 108 compresses the UDP/IP/Ethernet headers into a light-weight header by removing static information that is already known to the AP.
(3) The high level packet is fragmented into a number of smaller packets for transmission across the DECT air link and is merged with any local control data. This fragmented packet information is then passed across a serial link between the portable device main board and the PWD radio module 107.
(4) The individual data packets are recovered by the PWD radio module 107 and control information that is to be used locally is then extracted. The data packets are then passed over the air to the access point through the DECT stack. The "ARQ" layer provides a retry mechanism for error correction.
(5) At the Access Point 110, the small DECT packets are combined to form the network packet. The lightweight header is then expanded back into the full UDP/IP and Ethernet MAC headers. The whole packet is then encapsulated into a Harmony UDP/IP frame and passed to the APC. Control data can also be exchanged between the APC and AP using the complaint UDP/IP frames.
(6) At the Access Point Controller (APC) 115, the packet is de-encapsulated and passed to the remote end point 120, via standard LAN protocols.
(7) The remote end point receives the data and uses it as required. From the perspective of the remote end point, the data appears to have originated from a portable device which is attached to the wired network.

It should be noted that when transmitting in the opposite direction from the PIC to the portable device, the protocol functions in the same manner.

At the time of manufacture, the device has a unique Ethernet address allocated to each radio module and each access point. The radio module (or access point) then uses a mapping rule, such as using the low 20 bits of the Ethernet MAC address to calculate its unique radio identity. The Ethernet MAC address is registered in a normal way. The radio identity must be unique among all radio devices manufactured, but does not need to be registered with an external body.

Conventional WMTS is purely a network transport mechanism, and does not have any knowledge of the type of data being passed. All of the knowledge regarding the type of data passed is held by the applications running at either end point on the link. WMTS network transport is primarily intended to support IP protocols, as defined in STD 5 OF IEEE.

With regard to Dynamic data, the AP caches dynamic data that primarily allows it to resolve IP addresses into Ethernet address (i.e. routing tables). This information is passed between the APs during hand over, and could be corrupted during transmission. If this data cache is lost or corrupted hen its contents can be refreshed from the network. The impact on WMTS is that data may be delayed while address resolution takes place. However, the fact that the wired networked is approximately 10,000 times faster than the wireless network means that this delay will have minimal or no impact at all on system performance.

Fig. 2A and 2B illustrate an extended data protocol model according to the present invention. Here, the portable device 205 communicates with a remote client 220 via a protocol stack 201 that includes a PWD radio module 207, a WMTS Access Point 210 and a Standard Access Point Controller 215.

WMTS Access Point 210 includes a Gateway Translation Function (GTF) module 212 that is required to provide format translations functions between a local protocol 212a of a device, and a standard protocol, for example IEEE 1073. Thus, the local protocol communicates with the device, and the standard protocol is used for communication in the network. Alternatively, with standardization, the local protocol may be replaced with a Standard WMTS AP protocol. The WMTS Access Point 210 typically receives information from the PWD via DECT, and communicates with the remote client 220 via Ethernet, which is typically wired Ethernet. However, the present invention is not limited to wired Ethernet, or a wired network, and totally wireless network could be used, and/or wireless Ethernet. It is envisioned that there may be WLANs with old-style PWDs that provide emulation and much lighter, smaller and less expensive PWDs that have their emulation performed by the AP.

The Gateway Translation Function Module 212 according to the present invention emulates the upper layered functions of the network so that Ethernet packets can be sent from the PWD to the remote client end-to-end without the PWD devices being loaded down with emulation capabilities. The prior art PWD devices needed to emulated transmission into a standard protocol for transmission through standard WLAN-LAN bridges. For the most part, standard WLAN-LAN bridges merely allow the information to pass through without any manipulation/conversion.

An advantage of the present invention is that the local PWD devices do not need as much bandwidth to emulate the upper layer Ethernet functions because the GTF will convert their protocol into standard protocol at the WMTS Access Point. Nor do the local devices need as much power as the complexity of their transmission is reduced. Thus, the WMTS Access Point of the present invention operates as a Virtual Medical Device that performs the emulation instead of having every single PWD device have such capabilities engineered into them.

The standard protocol in the GTF may comprise or be based on a bona fide medical device industry standard in the case of wireless medical devices communicating with a remote LAN via, for example, DECT, to an emulating AP. A less costly alternative is also to provide Ethernet-Internet compatible low-end telemetric device bridging capability, where for example, the device may communicate using TCP/IP and the network uses Ethernet.

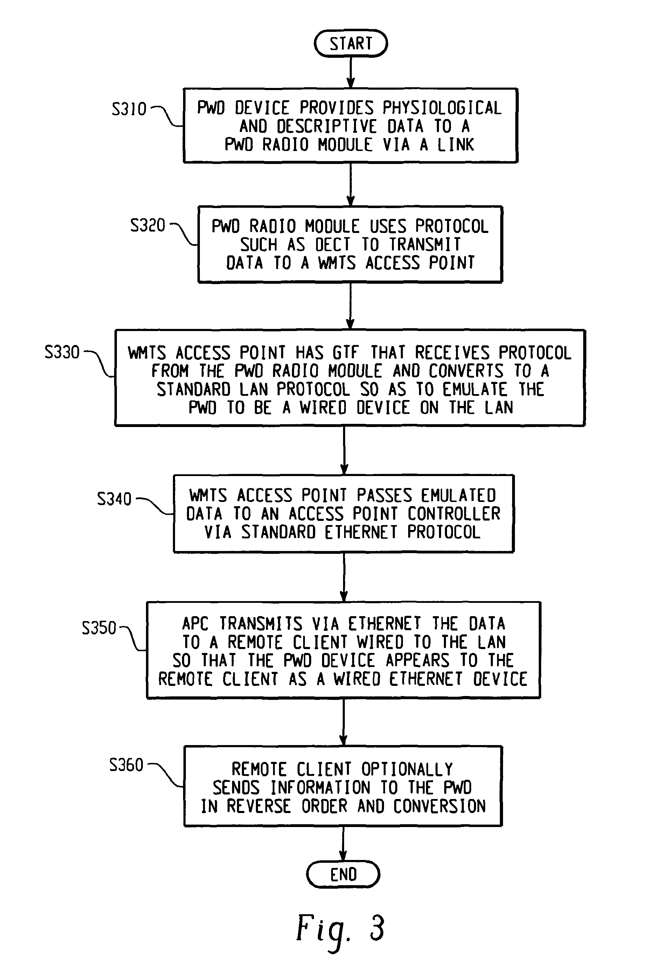

Fig. 3 illustrates a method of operation according to the present invention. It should be understood that certain protocols, such as DECT and Ethernet, are chosen for explanatory purposes and the claimed invention is in no way limited to such protocols or equivalents thereof, as any type of WLAN-LAN communication benefits from the advantages of the present invention. First, a PWD device periodically transmits (via a PWD radio module) physiological data in packets according to a local protocol, such as DECT transmissions via TDMA. Second, the information is received by a WMTS Access Point have a GTF module therein. At the GTF module, the local protocol is emulated (i.e. converted) into a standard protocol such as IEEE 802.3. Third, the Access Point controller then provides the information to a remote client (such as a central site of a hospital) via Ethernet protocol. Thus, a WLAN to wired LAN transmission conversion has been made, as to the remote client, it appears that the PWD is a wired device on the network transmitting under standard Ethernet protocols.

The present invention also offers advantage in terms of scalability and increased through-put, as the burdens of emulation are removed from the devices. In the field of wireless medical telemetry, as the costs per PWD device go down, the greater will be the ability to afford to medically monitor more than just hospital/hospice patients. For example, anyone with a heart problem could potentially have a PWD device that would periodically transmit information to a remote network, and in the case that the physiological responses were out of range, anything from beeping to the patient to call their doctor to dispatching an ambulance is possible. However, so long as individual PWD device are required to emulate high level network capabilities to communicate with a remote site, this goal would remain elusive. The present invention provides a means to reduce the size, power consumption and bandwidth use of PWDs by off shifting the emulation to another part of the network, typically the Access Point.

Various modifications may be made to the present invention by a person of ordinary skill in the art that lies within the spirit of the invention and the scope of the appended claims. For example, the transmission standards, such as DECT, could be substituted for a different standard according to need. The TDMA transmission could be replaced with CDMA, GSM, FMDA, etc. The PWD could, for example, use Bluetooth, or a version of 802.11, may or may not operate on UWB frequencies. The remote client is typically wired via Ethernet cable, such as Cat-5/Cat-5 (or another category according to need), but the network could be a fiber optic network that does not employ Ethernet, or for some unknown reason, uses Token Ring. The standards and protocols discussed were provided in accordance with a preferred operation of the invention, but do not limit the claimed invention to only those disclosed.

## Claims

1. A method for WMTS/DECT medical monitoring system comprising an Access Point to emulate a standard transmission protocol used in a Local Area Network so as to permit Wireless Local Area Network devices to communicate with devices wired to a Local Area Network, comprising the steps of:
(a) transmitting by at least one wireless device (205) at least a portion of a data packet to an Access Point (210) of a Local Area Network LAN , wherein said at least one wireless device (205) transmits according to a wireless local protocol (212a) and does not emulate a standard protocol (212b) of the Local Area Network LAN , wherein the at least one wireless device (205) has a unique Ethernet address allocated thereto, and wherein the at least one wireless device (205) or an emulation Access Point (210) uses a mapping rule to calculate a unique radio identity of the at least one wireless device (205) from said unique Ethernet address;
(b) receiving the transmission from the wireless device (205) by the emulation Access Point (210) comprising a Gateway Function Translation module (212), the emulation Access Point (210) providing said at least a portion of a data packet to the Gateway Function Translation module (212);
(c) converting of said at least a portion of the data packet transmitted by the wireless device (205) in the wireless local protocol (212a) by the Gateway Function Translation module (212) to the standard protocol (212b) of the Local Area Network LAN , wherein the emulation Access Point (210) forwards the at least a portion of the data packet converted to the standard protocol (212b) to an Access Point Controller (215); and
(d) providing by the Access Point Controller (215) said at least a portion of the data packet to a remote client (220) via the standard protocol (212b), said Access Point Controller (215) emulating sending the data to the remote client (220) via the standard protocol (212b) so that the remote client responds (220) as if the at least a portion of the data packet sent by the wireless device (205) appears to be from a wired device on the Local Area Network LAN.

2. The method according to claim 1, wherein the at least one wireless device recited in step (a) comprises a Patient Wearable Device PWD for monitoring physiological responses of a patient.

3. The method according to claim 2, wherein the Patient Wearable Device PWD comprises a heart monitor.

4. The method according to claim 2, wherein the Patient Wearable Device PWD comprises a blood pressure monitor.

5. The method according to claim 1, wherein the wireless local protocol (212a) comprises a Wireless Medical Telemetry System WMTS/ Digital Enhanced Cordless Telecommunications DECT standard of wireless transmission.

6. The method according to claim 1, wherein the wireless local protocol (212a) comprises an IEEE 802.11 wireless protocol.

7. The method according to claim 1, wherein the standard protocol (212b) comprises Ethernet protocol.

8. The method according to claim 1, wherein the standard protocol (212b) comprises fiber optic communication protocol.

9. The method according to claim 1, wherein the standard protocol (212b) comprises an IEEE 1073 protocol.

10. An emulation Access Point (210) for providing an Intermediate Distribution Frame IDF for transmission between a wireless device (205) and a remote client (220) on a wired Local Area Network LAN, wherein a protocol stack (201) comprises the Access Point (210) adapted for communication with the radio module (207) of the wireless device and an Access Point Controller (215); **characterized in that** the Access Point (210) comprises a Gateway Translation function module (212) that is adapted for receiving data transmitted in a wireless local protocol (212a) from the wireless device radio module (207) of the wireless device (205), without a standard protocol (212b) of the Local Area Network (LAN) being emulated by the wireless device (205), and translating the data to the standard protocol (212b) that is transmitted to the remote client, wherein the wireless device (205) has a unique Ethernet address allocated thereto, and wherein the Access Point (210) uses a mapping rule to calculate a unique radio identity of the wireless device (205) from said unique Ethernet address.

11. The emulation Access Point according to claim 10, wherein the Gateway Translation function module (212) translates the wireless local protocol (212a) into an Ethernet protocol or an IEEE 1073 protocol.

12. The emulation Access Point according to claim 10, wherein the wireless local protocol (212a) comprises Wireless Medical Telemetry/Digital Enhanced Cordless Telecommunications WMTS/DECT or wireless transmissions according to IEEE 802.11.

13. A WMTS/DECT medical monitoring system that is adapted for a Wireless-LAN communicating with a wired-LAN, comprising:
a plurality of wireless patient wearable devices PWD is adapted to monitor a physiological response of a patient;
at least one or more of the plurality of patient wearable devices (PWD) having a respective PWD radio module for transmitting/receiving data, wherein the patient wearable devices PWD are adapted to transmit using a wireless local protocol (212a) through a Wireless Medical Telemetry System WMTS Emulation access Point to a remote client (220) that is wired to a LAN and is adapted to communicate via a standard protocol (212b) comprising one of an Ethernet protocol and IEEE 1073 medical protocol, wherein the PWD radio module is adapted to not emulate the standard protocol (212b); and the Wireless Medical Telemetry System WMTS emulation Access Point (210) for receiving the local protocol (212a) transmissions from said at least one or more PWD radio modules, the Access Point (210) further comprising a Gateway Translation Function Module (212) that is adapted to translate the wireless local protocol (212a) used by the PWD radio modules into the standard protocol (212b) used by the remote client 220,
wherein at least one patient wearable device has a unique Ethernet address allocated thereto, and wherein the at least one patient wearable device or the Access Point (210) uses a mapping rule to calculate a unique radio identity of the at least one patient wearable device from said unique Ethernet address.

14. The system according to claim 13, wherein the wireless local protocol (212a) transmitted by the patient wearable devices PWD comprises a WMTS/DECT standard or an IEEE 802.11 wireless protocol.

15. The system according to claim 14, wherein wireless transmissions utilize an Ultra-Wide Band UWB frequency.

## Patentansprüche

1. Verfahren für ein medizinische WMTS/DECT-Überwachungssystem, umfassend einen Zugriffspunkt (engl. access point) zum Emulieren eines in einem lokalen Netzwerk (engl. local area network, LAN) verwendeten Standardübertragungsprotokolls, um so Geräten in einem drahtlosen lokalen Netzwerk (engl. wireless local area network, WLAN) zu erlauben, mit den mit einem lokalen Netzwerk drahtgebunden verbundenen Geräten zu kommunizieren, wobei das Verfahren die folgenden Schritte umfasst:
(a) Senden von mindestens einem Teil eines Datenpakets an einen Zugriffspunkt (210) eines lokalen Netzwerks LAN durch mindestens ein drahtloses Gerät (205), wobei das genannte mindestens eine drahtlose Gerät (205) gemäß einem drahtlosen lokalen Protokoll (212a) sendet und nicht ein Standardprotokoll (212b) des lokalen Netzwerks LAN emuliert, wobei dem mindestens einen drahtlosen Gerät (205) eine eindeutige Ethernet-Adresse zugeordnet ist, und wobei das mindestens eine drahtlose Gerät (205) oder ein Emulations-Zugriffspunkt (210) eine Mapping-Regel verwendet, um eine eindeutige Funkidentität des mindestens einen drahtlosen Geräts (205) anhand der eindeutigen Ethernet-Adresse zu berechnen;
(b) Empfangen der Übertragung von dem drahtlosen Gerät (205) durch den Emulations-Zugriffspunkt (210) umfassend ein Gateway-Function-Translation-Modul (212), wobei der Emulations-Zugriffspunkt (210) dem Gateway-Function-Translation-Modul (212) mindestens einen Teil eines Datenpakets bereitstellt;
(c) Umwandeln des genannten mindestens Teils des Datenpakets, das durch das drahtlose Gerät (205) im drahtlosen lokalen Protokoll (212a) gesendet wurde, durch das Gateway-Function-Translation-Modul (212) in das Standardprotokoll (212b) des lokalen Netzwerks LAN, wobei der Emulations-Zugriffspunkt (210) den in das Standardprotokoll (212b) umgewandelten mindestens einen Teil des Datenpakets an eine Zugriffspunkt-Steuereinheit (engl. access point controller) (215) weiterleitet; und
(d) Bereitstellen des genannten mindestens einen Teils des Datenpakets durch die Zugriffspunkt-Steuereinheit (215) für einen abgesetzten Client (220) über das Standardprotokoll (212b), wobei die Zugriffspunkt-Steuereinheit (215) das Senden der Daten an den abgesetzten Client (220) über das Standardprotokoll (212b) emuliert, so dass der abgesetzte Client (220) so reagiert, als stamme der durch das drahtlose Gerät (205) gesendete mindestens eine Teil des Datenpakets von einem verdrahteten Gerät im lokalen Netzwerk LAN.

2. Verfahren nach Anspruch 1, wobei das in Schritt (a) genannte mindestens eine drahtlose Gerät ein vom Patienten tragbares Gerät (engl. patient wearable device, PWD) zum Überwachen von physiologischen Reaktionen eines Patienten umfasst.

3. Verfahren nach Anspruch 2, wobei das vom Patienten tragbare Gerät PWD einen Herzmonitor umfasst.

4. Verfahren nach Anspruch 2, wobei das vom Patienten tragbare Gerät PWD einen Blutdruckmonitor umfasst.

5. Verfahren nach Anspruch 1, wobei das drahtlose lokale Protokoll (212a) einen WMTS- (Wireless Medical Telemetry System, drahtloses medizinisches Telemetriesystem) / DECT- (Digital Enhanced Cordless Telecommunications) Standard oder drahtlose Übertragung umfasst.

6. Verfahren nach Anspruch 1, wobei das drahtlose lokale Protokoll (212a) ein drahtloses Protokoll nach IEEE 802.11 umfasst.

7. Verfahren nach Anspruch 1, wobei das Standardprotokoll (212b) ein Ethernet-Protokoll umfasst.

8. Verfahren nach Anspruch 1, wobei das Standardprotokoll (212b) ein faseroptisches Kommunikationsprotokoll umfasst.

9. Verfahren nach Anspruch 1, wobei das Standardprotokoll (212b) ein Protokoll nach IEEE 1073 umfasst.

10. Emulations-Zugriffspunkt (210) zum Bereitstellen eines Zwischenverteilungsrahmens (engl. intermediate distribution frame, IDF) zur Übertragung zwischen einem drahtlosen Gerät (205) und einem abgesetzten Client (220) auf einem verdrahteten lokalen Netzwerk LAN, wobei ein Protokollstapel (201) den Zugriffspunkt (210) umfasst, der für die Kommunikation mit dem Funkmodul (207) des drahtlosen Geräts und einer Zugriffspunkt-Steuereinheit (215) ausgelegt ist,
**dadurch gekennzeichnet, dass**
der Zugriffspunkt (210) ein Gateway-Translation-Function-Modul (212) umfasst, welches dafür ausgelegt ist, in einem drahtlosen lokalen Protokoll (212a) von dem Drahtlosgerät-Funkmodul (207) des drahtlosen Geräts (205) gesendete Daten zu empfangen, ohne dass ein Standardprotokoll (212b) des lokalen Netzwerks (LAN) durch das drahtlose Gerät (205) emuliert wird, und die Daten in das Standardprotokoll (212b) umzusetzen, das an den abgesetzten Client gesendet wird,
wobei dem drahtlosen Gerät (205) eine eindeutige Ethernet-Adresse zugeordnet ist, und
wobei der Zugriffspunkt (210) eine Mapping-Regel verwendet, um eine eindeutige Funkidentität des mindestens des drahtlosen Geräts (205) anhand der eindeutigen Ethernet-Adresse zu berechnen.

11. Emulations-Zugriffspunkt nach Anspruch 10, wobei das Gateway-Translation-Function-Modul (212) das drahtlose lokale Protokoll (212a) in ein Ethernet-Protokoll oder ein IEEE 1073-Protokoll umsetzt.

12. Emulations-Zugriffspunkt nach Anspruch 10, wobei das drahtlose lokale Protokoll (212a) Wireless Medical Telemetry/Digital Enhanced Cordless Telecommunications WMTS/DECT- oder drahtlose Übertragung nach IEEE 802.11 umfasst.

13. Medizinisches WMTS/DECT-Überwachungssystem, das für eine WLAN-Kommunikation mit einem verdrahteten LAN ausgelegt ist, umfassend:
eine Vielzahl von drahtlosen, von einem Patienten tragbaren Geräten PWD, die dafür ausgelegt ist, eine physiologische Reaktion eines Patienten zu überwachen;
wobei mindestens eines oder mehrere der Vielzahl der von dem Patienten tragbaren Geräte (PWD) ein jeweiliges PWD-Funkmodul zum Senden/Empfangen von Daten aufweisen, wobei die vom Patienten tragbaren Geräte PWD für das Senden unter Verwendung eines drahtlosen lokalen Protokolls (212a) über einen WMTS-Emulations-Zugriffspunkt an einen abgesetzten Client (220) ausgelegt sind, der mit einem LAN drahtgebunden verbunden ist und dafür ausgelegt ist, über ein Standardprotokoll (212b) umfassend entweder ein Ethernet-Protokoll oder ein medizinisches IEEE 1073-Protokoll zu kommunizieren, wobei das PWD-Funkmodul dafür ausgelegt ist, nicht das Standardprotokoll (212b) zu emulieren; und
den WMTS-Emulations-Zugriffspunkt (210) zum Empfangen der Übertragungen über das lokale Protokoll (212a) von den genannten mindestens einen oder mehreren PWD-Funkmodulen, wobei der Zugriffspunkt (210) weiterhin ein Gateway-Translation-Function-Modul (212) umfasst, das dafür ausgelegt ist, das von den PWD-Funkmodulen verwendete drahtlose lokale Protokoll (212a) in das durch den abgesetzten Client (220) verwendete Standardprotokoll (212b) umzusetzen,
wobei mindestens einem vom Patienten tragbaren Gerät eine eindeutige Ethernet-Adresse zugewiesen ist, und wobei das mindestens eine vom Patienten tragbare Gerät oder der Zugriffspunkt (210) eine Mapping-Regel verwendet, um eine eindeutige Funkidentität für das mindestens eine vom Patienten tragbare Gerät anhand der genannten eindeutigen Ethernet-Adresse zu berechnen.

14. System nach Anspruch 13, wobei das durch die vom Patienten tragbaren Geräte PWD übertragene drahtlose lokale Protokoll (212a) ein WMTS/DECT-Standardprotokoll oder ein Drahtlos-Protokoll nach IEEE 802.11 umfasst.

15. System nach Anspruch 14, wobei die drahtlosen Übertragungen eine Ultrabreitband- (UWB) Frequenz nutzen.

## Revendications

1. Procédé pour un système de surveillance médicale WMTS/DECT comprenant un point d'accès pour émuler un protocole de transmission standard utilisé dans un réseau local de manière à permettre à des dispositifs de réseau local sans fil de communiquer avec des dispositifs câblés à un réseau local, comprenant les étapes de :
(a) la transmission, par au moins un dispositif sans fil (205), d'au moins une portion d'un paquet de données à un point d'accès (210) d'un réseau local, LAN, dans lequel ledit au moins un dispositif sans fil (205) transmet selon un protocole local sans fil (212a) et n'émule pas un protocole standard (212b) du réseau local, LAN, dans lequel l'au moins un dispositif sans fil (205) a une adresse Ethernet unique qui lui est allouée, et dans lequel l'au moins un dispositif sans fil (205) ou un point d'accès d'émulation (210) utilise une règle de mappage pour calculer une identité radio unique de l'au moins un dispositif sans fil (205) à partir de ladite adresse Ethernet unique ;
(b) la réception de la transmission à partir du dispositif sans fil (205) par le point d'accès d'émulation (210) comprenant un module de traduction de fonction de passerelle (212), le point d'accès d'émulation (210) fournissant ladite au moins une portion d'un paquet de données au module de traduction de fonction de passerelle (212) ;
(c) la conversion de ladite au moins une portion du paquet de données transmise par le dispositif sans fil (205) dans le protocole local sans fil (212a) par le module de traduction de fonction de passerelle (212) au protocole standard (212b) du réseau local, LAN, dans lequel le point d'accès d'émulation (210) transfère l'au moins une portion du paquet de données convertie dans le protocole standard (212b) à un contrôleur de point d'accès (215) ; et
(d) la fourniture, par le contrôleur de point d'accès (215), de ladite au moins une portion du paquet de données à un client distant (220) par l'intermédiaire du protocole standard (212b), ledit contrôleur de point d'accès (215) émulant l'envoi des données au client distant (220) par l'intermédiaire du protocole standard (212b) de sorte que le client distant réponde (220) comme si l'au moins une portion du paquet de données envoyée par le dispositif sans fil (205) semblait provenir d'un dispositif câblé au réseau local, LAN.

2. Procédé selon la revendication 1, dans lequel l'au moins un dispositif sans fil selon l'étape (a) comprend un dispositif portable par patient, PWD, pour surveiller des réponses physiologiques d'un patient.

3. Procédé selon la revendication 2, dans lequel le dispositif portable par patient, PWD, comprend un moniteur cardiaque.

4. Procédé selon la revendication 2, dans lequel le dispositif portable par patient, PWD, comprend un moniteur de pression sanguine.

5. Procédé selon la revendication 1, dans lequel le protocole local sans fil (212a) comprend une norme de système de télémétrie médicale sans fil, WMTS/télécommunications sans fil améliorées numériques, DECT, ou des transmissions sans fil.

6. Procédé selon la revendication 1, dans lequel le protocole local sans fil (212a) comprend un protocole sans fil IEEE 802.11.

7. Procédé selon la revendication 1, dans lequel le protocole standard (212b) comprend un protocole Ethernet.

8. Procédé selon la revendication 1, dans lequel le protocole standard (212b) comprend un protocole de communication à fibre optique.

9. Procédé selon la revendication 1, dans lequel le protocole standard (212b) comprend un protocole IEEE 1073.

10. Point d'accès d'émulation (210) pour fournir une trame de distribution intermédiaire, IDF, pour une transmission entre un dispositif sans fil (205) et un client distant (220) sur un réseau local, LAN, câblé, dans lequel une pile de protocoles (201) comprend le point d'accès (210) apte à communiquer avec le module radio (207) du dispositif sans fil, et un contrôleur de point d'accès (215) ;
**caractérisé en ce que**
le point d'accès (210) comprend un module de fonction de traduction de passerelle (212) qui est apte à recevoir des données transmises dans un protocole local sans fil (212a) à partir du module radio de dispositif sans fil (207) du dispositif sans fil (205), sans qu'un protocole standard (212b) du réseau local, LAN, ne soit émulé par le dispositif sans fil (205), et à traduire les données dans le protocole standard (212b) qui sont transmises au dispositif client,
dans lequel le dispositif sans fil (205) a une adresse Ethernet unique qui lui est allouée, et
dans lequel le point d'accès (210) utilise une règle de mappage pour calculer une identité radio unique du dispositif sans fil (205) à partir de ladite adresse Ethernet unique.

11. Point d'accès d'émulation selon la revendication 10, dans lequel le module de fonction de traduction de passerelle (212) traduit le protocole local sans fil (212a) en un protocole Ethernet ou un protocole IEEE 1073.

12. Point d'accès d'émulation selon la revendication 10, dans lequel le protocole local sans fil (212a) comprend une norme de système de télémétrie médicale sans fil, WMTS/télécommunications sans fil améliorées numériques, DCT, ou des transmissions sans fil selon IEEE 802.11.

13. Système de surveillance médicale WMTS/DECT qui est apte à effectuer une communication d'un réseau local, LAN, sans fil à un réseau local, LAN, câblé, comprenant :
une pluralité de dispositifs portables par patient, PWD, sans fil aptes à surveiller une réponse physiologique d'un patient ;
au moins un ou plusieurs de la pluralité de dispositifs portables par patient, PWD, comportant un module radio PWD respectif pour transmettre/recevoir des données, dans lequel les dispositifs portables par patient, PWD, sont aptes à transmettre en utilisant un protocole local sans fil (212a) par l'intermédiaire d'un point d'accès d'émulation de système de télémétrie médicale sans fil, WMTS, à un client distant (220) qui est câblé à un LAN et qui est apte à communiquer par l'intermédiaire d'un protocole standard (212b) comprenant l'un d'un protocole Ethernet et d'un protocole médical IEEE 1073, dans lequel le module radio PWD est apte à ne pas émuler le protocole standard (212b) ; et
le point d'accès d'émulation de système de télémétrie médicale sans fil, WMTS, (210) pour recevoir les transmission de protocole local (212a) à partir desdits au moins un ou plusieurs modules radio PWD, le point d'accès (210) comprenant en outre un module de fonction de traduction de passerelle (212) qui est apte à traduire le protocole local sans fil (212a) utilisé par les modules radio PWD dans le protocole standard (212b) utilisé par le client distant (220),
dans lequel au moins un dispositif portable par patient a une adresse Ethernet unique qui lui est allouée, et dans lequel l'au moins un dispositif portable par patient ou le point d'accès (210) utilise une règle de mappage pour calculer une identité radio unique de l'au moins un dispositif portable par patient à partir de ladite adresse Ethernet unique.

14. Système selon la revendication 13, dans lequel le protocole local sans fil (212a) transmis par les dispositifs portables par patient, PWD, comprend une norme WMTS/DECT ou un protocole sans fil IEEE 802.11.

15. Système selon la revendication 14, dans lequel des transmissions sans fil utilisent une fréquence de bande ultralarge, UWB.
